(19) Europäisches Patentamt
European Patent Office
Office européen des brevets

(11) Publication number: 0 350 672 A1

# (12) EUROPEAN PATENT APPLICATION

(21) Application number: 89111279.9

(22) Date of filing: 21.06.89

(51) Int. Cl.[4]: C07F 9/10 , C07C 229/00 , A61K 31/66

(30) Priority: 28.06.88 IT 2112088
28.06.88 IT 2112188

(43) Date of publication of application:
17.01.90 Bulletin 90/03

(84) Designated Contracting States:
BE DE ES FR GB GR IT LU NL

(71) Applicant: MAGIS FARMACEUTICI S.p.A.
Via Cacciamali 34/36/38
I-25125 Brescia(IT)

(72) Inventor: Puricelli, Laura
Via Taramelli 7
I-25100 Brescia(IT)

(74) Representative: Gervasi, Gemma et al
NOTARBARTOLO & GERVASI Srl Viale
Bianca Maria 33
I-20122 Milan(IT)

## (54) Salts of diacyl-glycero-(di)-phosphates of L-(acyl) carnitine and of L-acyl-carnitine esters.

(57) The α-glycero (di) phosphate di-O-acyl derivatives of L-carnitine and its esters and of L-acylcarnitine and its esters are described.

These new acyl derivatives are usefyl in the treatment of cardiac disturbances, dyslipemias, slow cerebral metabolism, senile and pre-senile psychomotor involution, depressive states, senile and pre-senile dementia, reduced cerebral blood flow and the sequelae of primitive cerebral ictus.

EP 0 350 672 A1

# SALTS OF DIACYL-GLYCERO-(DI)-PHOSPHATES OF L-(ACYL) CARNITINE AND OF L-ACYL-CARNITINE ESTERS.

## SUMMARY OF THE INVENTION

This invention relates to the salt of L-carnitine and its esters, and of L-acylcarnitine and its esters, with the di-acyl derivative of $\alpha$-glycero-(di)-phosphoric acid in its D, L or DL form, a process for their preparation and pharmaceutical compositions which contain them.

More particularly, the invention relates to the compounds of the following general formula (I):

$CH_2$-OR
CH-OR$'$
$CH_2$-OY (I)
wherein

Y =

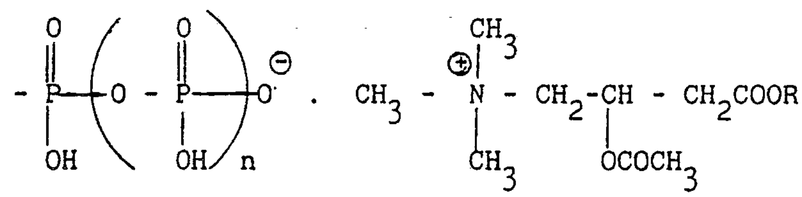

wherein n can be 0 or 1
where R and R$'$ are identical or different radicals of a saturated or unsaturated $C_3$-$C_{22}$ acid, $R_2$ is H or a saturated or unsaturated $C_2$-$C_{18}$ acid radical, and $R_3$ is H or a $C_1$-$C_6$ alkyl radical.

## DETAILED DESCRIPTION

The characteristics and advantages of the present invention will be more apparent from the following detailed description of the process for preparing the derivatives of general formula (I), their characteristics and the pharmaceutical compositions which contain them.

The derivatives of formula (I) wherein n = 0 are prepared by a process consisting of reacting equivalent quantities of L-carnitine or its ester, or of L-acylcarnitine or its ester, in ethanolic solution with 1,2-di-O-acyl glycerophosphoric acid.

The compound obtained is isolated by adding ace tone and eliminating the ethanol under vacuum.

The 1,2-di-O-acyl glycerophosphoric acid is obtained by reacting glycerophosphoric acid with an acyl chloride or acyl anhydride, or a mixture of acyl chlorides or acyl anhydrides of formula (II): R (R$'$) COX wherein X = Cl or R (R$'$) COO-wherein R and R$'$ have the above mentioned meanings.

The derivatives of formula (I) wherein n = 1 are prepared by a process consisting of reacting equivalent quantities of L-carnitine or its ester, or of L-acyl carnitine or its ester, in ethanolic solution with 1,2-di-O-acyl glycerodiphosphoric acid.

The compound obtained is isolated by adding acetone and evaporating the ethanol under vacuum.

The 1,2-di-O-acyl glycerodiphosphoric acid is obtained by reacting 1,2-di-O-acyl glycerophosphoric acid above mentioned with 85% phosphoric acid in a mixture of water and pyridine in the presence of dicyclohexylcarbodiimide (D.C.C.).

The derivatives according to the present invention are extremely effective therapeutic agents in the treatment of slow cerebral metabolism, senile and pre-senile psychomotor involution, depressive states, senile and pre-senile dementia, reduced cerebral blood flow, the sequelae of primitive cerebral ictus, and cardiac affections.

The present invention therefore also relates to pharmaceutical compositions containing a therapeutically active quantity of active principle of general formula (I) dissolved in and/or mixed with a pharmaceutically acceptable liquid or solid support normally used for these compositions.

The compositions of the invention can be administered orally or by intramuscular injection.

For oral administration a non-toxic pharmaceutical composition can be prepared by mixing the active

substance with the supports used for this purpose, such as starch, glucose, lactose, gelatin, magnesium stearate, glyceryl monostearate, talc, sodium chloride, propyleneglycol, ethanol, olive oil etc. The final products are therefore in hard or soft capsule tablet, pill, single-dose sachet or extemporaneous suspension or emulsion form.

In the case of injectable formulations, the active principles are dissolved or suspended in liquids suitable for forming vials.

In the treatment of slow cerebral metabolism, senile and presenile psychomotor involution, depressive states, senile and pre-senile dementia, reduced cerebral blood flow, the sequelae of primitive cerebral ictus, cardiac affections, dyslipemias and hyperlipoproteinemias, the compounds according to the invention can be administered in quantities ranging from 5 to 50 mg/kg per day in doses separated in time, such as three or four times per day.

The pharmaceutical compositions of the invention can be administered in posological units containing for example 250, 500, 1000 and 1500 mg of active principle together with physiologically suitable supports and excipients.

The pharmaco-biological characteristics of the compounds according to the present invention were evaluated with particular reference to acute toxicity, their effects in terms of modifying the lipoprotein and hyper-excitability states and their capacity to return cholesterol and triglyceride levels to normal values.

Said compounds have low toxicity and high activity.

The acute toxicity of the compounds of general formula (I) was studied on the mouse by the Weal method (C.S. Weal, Biomed. J. 249, 1952).

The $LD_{50}$ values of the compounds of general formula (I) are around 3000 mg/kg.

The cardiokinetic effect was studied on a Langerdoff isolated rabbit heart preparation.

The tests show that said compounds have a positive inotropic effect calculated 10 minutes after interrupting the anoxic period.

The antiarrhythmic effect of the new compounds was studied on the mouse by the procedure of P.w. Wangow and T. Holcow (P.W.N. Wangow, T.L. Holcow, Arch. Int. Pharmacodin. 224, 219, 1977). The results show that said compounds have a beneficial effect on arrhythmia induced by aconitine (5 $\gamma$/ml).

The effect of the compounds according to the invention in terms of modifying the lipoprotein state and the cholesterol and triglyceride plasmatic levels, altered by oral administration of olive oil, was studied in rats by administering 300 mg/kg of said compounds after orally administering 15 mg/kg of olive oil.

The compounds proved able to return the triglyceride, cholesterol and the $\alpha$-$\beta$ and pre-$\beta$ lipoprotein levels to normal values.

The activity of the new compounds on the hyperexcitability state and on clonic contractions followed by depression induced by injection into the cerebellomedullary cistern of rats was studied by the method described by Irwing B. Frity in "Carnitine and its role in fatty acid metabolism" in "Advances in lipid research" I, 285-234, (1963) Academic Press. Intracerebroventricular administration of the new compounds causes pupil dilation and markedly increased activity.

The following examples illustrate the invention but do not limit it.

EXAMPLE 1

1,2-di-O-palmitoyl glycerophosphoric acid

A mixture containing 17.208 g of $\alpha$-glycerophosphoric acid, 70 ml of anhydrous pyridine and 105 g of palmitic anhydride is left stirring for 24 hours.

The mixture is then poured into 200 ml of water containing ice and the pyridine and water are evaporated under vacuum in a rotary flask.

The residue is taken up with a mixture of ether and acetone, the supernatant liquid is removed by decantation and dried in an oven.

About 60 g of product are obtained.

Spectrographic analyses confirm the structure.

| Elementary analysis | C | H | P |
|---|---|---|---|
| calculated %<br>found % | 64.68<br>64.7 | 10.856<br>10.9 | 4.765<br>4.7 |

M.W. 649.92

EXAMPLE 2

1,2-di-O-(palmitoyl, stearoyl, oleoyl, palmitoloyl, linoleoyl) glycerophosphoric acid

100 g of a mixture of acid chlorides (11.7% of palmitic acid chloride, 4% of stearic acid chloride, 8.6% of palmitoleic acid chloride, 9.8% of oleic acid chloride, 59% of linoleic acid chloride) are placed in a 500 ml flask fitted with a stirrer, reflux condenser and calcium chloride tube.

17.208 g of anhydrous glycerophosphoric acid are added to the mixture.

The mixture is left stirring for 16 hours at ambient temperature (the temperature rises to about 50 °C during the first hour).

400 ml of chloroform are added to the mixture, which is stirred and filtered if necessary.

The mixture is concentrated to a volume of about 80 ml under vacuum, the solution obtained is passed through a silica gel column and elution carried out with a chloroform-methanol (9:1) mixture.

The fraction containing the 1,2-di-O-acyl-glycerophosphate is separated and evaporated to dryness.

About 60 g of product are obtained.

Spectrographic analyses confirm the structure.

EXAMPLE 3

Using the procedure of Example 1, 1,2-di-O-acyl derivatives are obtained with a saturated or unsaturated $C_3C_{22}$ acyl radical.

EXAMPLE 4

Using the procedure of Example 2, 1,2-di-O-acyl derivatives are obtained in which the acyl consists of a mixture of saturated and unsaturated $C_3C_{22}$ acyl radicals in various proportions.

EXAMPLE 5

1,2-di-O-palmitoyl glycerophosphoric-L-carnitine salt

64.992 g of 1,2-di-O-palmitoyl glycerophosphoric acid obtained according to Example 1 are dissolved in 250 ml of absolute alcohol, and when fully dissolved, 16.20 g of L-carnitine are added while stirring.

Stirring is continued while 500 ml of acetone are added.

The precipitate which forms is filtered off and dried in an oven.

About 80 grams of product are obtained.

Spectrographic analyses confirm the structure.

| Elementary analysis | C | H | N | P |
|---|---|---|---|---|
| calculated %<br>found % | 61.82<br>61.9 | 10.564<br>10.9 | 1.7268<br>1.7 | 3.818<br>3.8 |

M.W. 811.117

EXAMPLE 6

Using the procedure of Example 5, the corresponding salts of L-carnitine esters are obtained having $C_1$-$C_6$ alkyl radical ($R^3$)

EXAMPLE 7

Using the procedure of Example 5, the salts of L-acetylcarnitine and its esters, and of L-acylcarnitine and its esters, are obtained by reacting the $\alpha$-glycerophosphoric acid prepared in Example 3.

EXAMPLE 8

Using the procedure of Example 5, the salts of L-carnitine and its esters, and of L-acylcarnitine and its esters are obtained by reacting 1,2-di-O-acyl glycerophosphoric acid prepared in Example 4.

EXAMPLE 9

Using the procedure of Example 5, the salts of L-carnitine and its esters, and of L-acylcarnitine and its esters, are obtained by reacting 1,2-di-O-acyl glycerophosphoric acid prepared in Example 3.

EXAMPLE 10

1,2-di-O-palmitoyl glycerodiphosphoric acid

6.499 g of 1,2-di-O-palmitoyl glycerophosphoric acid obtained according to Example 1 are mixed with 9.0 g of 85% phosphoric acid. The mixture is stirred and 30 ml of pyridine added followed by 80 g of D.C.C. (dicyclohexyl-carbodiimide). The mixture is stirred for 6 hours.

The dicyclohexylurea crystals which form are filtered off. They are taken up in chloroform which is then evaporated under vacuum in a rotary flask. The residue is taken up in 50 ml of water and repeated extraction is carried out with 50 ml fractions of chloroform.

The chloroform fractions are pooled and dried with anhydrous sodium sulphate, and then evaporated to dryness. The residue is washed with 50 ml of water and the pH adjusted to 7.0 with ammonia.

The solution is purified through a Dowex-2 (ion exchange resin) 200-325 mesh column in chloride form.

The column is washed with 20 ml of water to remove the pyridine, and the diphosphoric derivative then eluted, the separation being monitored by thin layer silica gel chromatography (eluent: chloroform-methanol 9: 1).

The fraction containing the diphosphoric derivative is saturated with sodium chloride and extracted repeatedly with chloroform fractions.

The chloroform layers are pooled and dried with anhydrous sodium sulphate and then evaporated to dryness. About 5 grams of the derivative are obtained.

Spectrographic analyses confirm the structure.

| Elementary analysis | C | H | P |
|---|---|---|---|
| calculated % | 57.515 | 9.93 | 8.474 |
| found % | 57.7 | 9.98 | 8.3 |

M.W. 730.91

EXAMPLE 11

Using the procedure of Example 10, 1,2-di-O-acyl derivatives of α-glycerodiphosphoric acid are obtained starting from the diacylglycerophosphoric acid of Example 3.

EXAMPLE 12

Using the procedure of Example 10, 1,2-di-O-acyl derivatives of α-glycerodiphosphoric acid are obtained starting from the di-acyl glycerophosphoric acids obtained in Example 4.

EXAMPLE 13

L-carnitine 1,2-di-O-palmitoyl glycerodiphosphate

73.091 g of 1,2-di-O-palmitoyl glycero diphosphoric acid obtained according to Example 10 are dissolved in 250 ml of absolute alcohol, and when fully dissolved, 16.27 g of L-carnitine are added while stirring.

Stirring is continued while 500 ml of acetone are added.

The precipitate which forms is filtered off and dried in an oven.

About 89 grams of product are obtained.

Spectrographic analyses confirm the structure.

| Elementary analysis | C | H | N | P |
|---|---|---|---|---|
| calculated % | 55.857 | 9.269 | 1.48 | 6.546 |
| found % | 56.0 | 9.4 | 1.45 | 6.48 |

M.W. 946.127

EXAMPLE 14

Using the procedure of Example 13, the corresponding salts of L-carnitine esters are obtained having a $C_1$-$C_6$ alkyl radical ($R^3$).

EXAMPLE 15

Using the procedure of Example 13, the salts of L-acetylcarnitine and its esters, and of L-acylcarnitine and its esters, are obtained by reacting the diacyl glycerodiphosphoric acid of Example 11.

EXAMPLE 16

Using the procedure of Example 13, the salts of L-carnitine and its esters, of L-acetylcarnitine and its esters, and of L-acylcarnitine and its esters, with 1,2-di-O-acyl diphosphoric acid are obtained by reacting the diacylglycerodiphosphoric acids of Example 12.

**Claims**

1. Salts of di-acyl-glycero (di) phosphates of L (acyl) carnitine and of L (acyl) carnitine esters having the general formula (I)

$CH_2$-OR
CH-OR′
$CH_2$-OY (I)

wherein

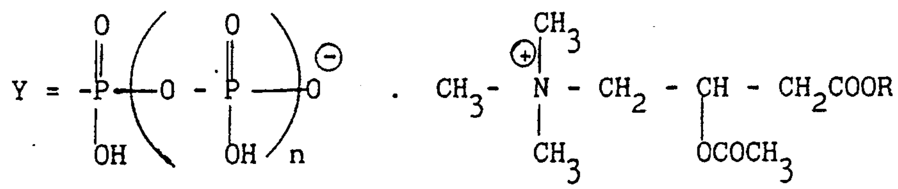

wherein n can be 0 or 1
where R and R′ are identical or different radicals of a saturated or unsaturated $C_3$-$C_{22}$ acid, $R_2$ is H or a saturated or unsaturated $C_2$-$C_{18}$ acid radical, and $R_3$ is H or a $C_1$-$C_6$ alkyl radical, said salts having the α-glycerophosphoric acid groups in D,L enantiomer or DL racemic form.

2. A process for preparing the compounds according to claim 1 comprising the following steps:

a) reacting the 1,2-glycerophosphoric acid with an acylchloride of anhydride or a mixture of acylchlorides or anhydrides having the formula
R (R′) COX (II) wherein X is Cl or R (R′) COO-,
wherein R and R′ have the above mentioned meanings.

b) directly salifying in alcohol the compounds obtained in step (a) with equivalent amounts of L-acylcarnitine and their esters for obtaining compounds having n = 0 or in the case of the compounds of formula (I) having n = 1;

c) the 1,2-diacylglycerophosphoric acid obtained in step (a) is reacted with $H_3PO_4$ in the presence of pyridine and dicyclohexylcarbodiimide and the obtained 1,2-di-O-acyl glycerodiphosphoric acid obtained is salified with equivalent amounts of carnitine, L-acyl-carnitine and their corresponding esters.

3. Pharmaceutical compositions for treating cardiac malfunctions, hyperlipoproteinemias, dyslipemias and slow cerebral metabolism, comprising a therapeutically effective quantity of the compound claimed in claim 1, in association with pharmacologically acceptable supports and adjuvants.

4. The pharmaceutical compositions according to claim 5, for oral or intramuscular administration.

5. The pharmaceutical compositions according to claim 5, containing from 250 to 1.500 mg of active principle.

6. A therapeutic method for the treatment of slow cerebral metabolism, senile and pre-senile dementia, reduced cerebral blood flow caused by primitive cerebral ictus, cerebral ischemia or cardiac affections by administering in stepped doses the pharmaceutical compositions according to claim 3, so that the contained active principle ranges from 5 to 50 mg/kg per day.

European Patent Office

# EUROPEAN SEARCH REPORT

Application Number

EP 89 11 1279

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-M- 6 345 (ETABLISSEMENTS FEVRIER, DECOISY, CHAMPION)<br>* Whole document *<br>--- | 1,3-6 | C 07 F 9/10<br>C 07 C 101/30<br>A 61 K 31/66 |
| A | EP-A-0 167 115 (MAGIS FARMACEUTICI S.p.A.)<br>* Whole document *<br>--- | 1,3-6 | |
| A | GB-A-2 045 612 (NIPPON SHOJI KAISHA LTD)<br>* Whole document *<br>----- | 1,3-6 | |
| | | | TECHNICAL FIELDS SEARCHED (Int. Cl.5) |
| | | | C 07 F 9/00<br>C 07 C 101/00<br>A 61 K 31/00 |

The present search report has been drawn up for all claims

| Place of search | Date of completion of the search | Examiner |
|---|---|---|
| THE HAGUE | 26-09-1989 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)